(19) Europäisches Patentamt
European Patent Office
Office européen des brevets

(11) EP 4 733 386 A1

(12) EUROPEAN PATENT APPLICATION
published in accordance with Art. 153(4) EPC

(43) Date of publication:
29.04.2026 Bulletin 2026/18

(21) Application number: 24825323.9

(22) Date of filing: 21.06.2024

(51) International Patent Classification (IPC):
C12N 1/20 (2026.01)     A23L 33/135 (2016.01)
A61K 35/745 (2015.01)     A61P 1/10 (2006.01)
C12P 19/04 (2006.01)     C12R 1/01 (2006.01)

(52) Cooperative Patent Classification (CPC):
A23L 33/135; A61K 35/745; A61P 1/10;
C12N 1/20; C12P 19/04; C12R 2001/01

(86) International application number:
PCT/CN2024/100538

(87) International publication number:
WO 2024/260436 (26.12.2024 Gazette 2024/52)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 21.06.2023 CN 202310744238
21.06.2023 CN 202310744483

(71) Applicant: COREE Company Limited
Hong Kong 999077 (HK)

(72) Inventors:
• WANG, Tingting
Beijing 101300 (CN)
• ZHANG, Shiqi
Beijing 101300 (CN)
• FAN, Linlin
Beijing 101300 (CN)
• ZHAO, Ying
Beijing 101300 (CN)
• DING, Yan
Beijing 101300 (CN)
• ZHANG, Di
Beijing 101300 (CN)
• LEE, Soowon
Beijing 101300 (CN)
• LIM, Chongyoon
Beijing 101300 (CN)

(74) Representative: Murgitroyd & Company
165-169 Scotland Street
Glasgow G5 8PL (GB)

Remarks:
The complete document including Reference Table(s) and the Sequence Listing(s) can be downloaded from the EPO website

(54) BIFIDOBACTERIUM ANIMALIS STRAIN, PROBIOTIC FORMULATION, AND PREPARATION METHOD FOR AND USE OF BIFIDOBACTERIUM ANIMALIS STRAIN

(57) The present invention relates to the technical field of microorganism, in particular to a *Bifidobacterium animalis* strain, a probiotic preparation, and a preparation method and use thereof. The deposit number of the *B. animalis* strain is CGMCC No. 25681. The *B. animalis* strain and the probiotic preparation thereof of the present invention can produce high levels of extracellular polysaccharides, lactic acid and short-chain fatty acids; sti-mulate mice to produce cytokines TNF-$\alpha$ and IL-6, thus have the function of enhancing immunity; and improve the ink propulsion rate, thus have a defecation effect. The single-strain powder of the *B. animalis* strain of the present invention can significantly enhance the cellular immune function and monocyte-macrophage function of mice.

EP 4 733 386 A1

FIG. 8

**Description**

**CROSS-REFERENCE TO RELATED APPLICATIONS**

This application claims priority to Chinese Patent Application No. 202310744238.4, filed June 21, 2023, and Chinese Patent Application No. 202310744483.5, filed June 21, 2023, the entire contents of which are incorporated herein by reference.

**TECHNICAL FIELD**

The present invention relates to the technical field of microorganisms, in particular to a *Bifidobacterium animalis* strain, a probiotic preparation, and a preparation method and use thereof.

**BACKGROUND**

Bifidobacterium is an important component of human intestinal flora, the number of which is closely related to human health conditions, and has many important physiological functions to human health, such as biological barrier, nutritional effect, anti-tumor effect, improvement of gastrointestinal function and anti-aging.

*Bifidobacterium animalis* is divided into two subspecies, which are *Bifidobacterium animalis* subsp. *animalis and Bifidobacterium animalis* subsp. *lactis,* which have the function of maintaining intestinal microecological balance and have good safety.

At present, the research on *B. animalis* and probiotic preparations thereof in improving immunity and defecation is still insufficient. Therefore, it is important to screen a strain of *B. animalis* subsp. *lactis* with excellent immune-enhancing and defecation performance.

**SUMMARY**

An object of the present invention is to provide a *Bifidobacterium animalis* (*B. animalis*) strain and a probiotic preparation thereof, wherein the strain and the probiotic preparation thereof can produce high levels of extracellular polysaccharides, lactic acid and short-chain fatty acids; improve the ink propulsion rate and have a defecation function.

Another object of the present invention is to provide a *B. animalis* strain and a probiotic preparation thereof, both of which can stimulate mouse macrophages RAW264.7 to produce cytokines TNF-$\alpha$ and IL-6, thereby improving immunity. The single-strain powder of *B. animalis* can significantly enhance the cellular immune function and monocyte-macrophage function of mouse, and the single-strain powder of *B. animalis* strain has excellent defecation function.

In order to achieve the above objects, the present invention provides the following technical solutions:

In one aspect, the present invention provides a *B. animalis* strain, wherein the deposit number of the *B. animalis* strain is CGMCC No. 25681.

Preferably, the *B. animalis* strain comprises a sequence of 16S rRNA gene represented by SEQ ID NO: 1.

The *B. animalis* strain of the present invention is derived from breast milk, can tolerate artificial gastrointestinal fluid, can adhere to intestinal epithelial cells, has a high survival rate in the intestine, and laying the foundation for its colonization and effective function in the intestine.

In one embodiment, the *B. animalis* strain has the ability to produce high levels of extracellular polysaccharides, lactic acid, and short-chain fatty acids, and can inhibit various pathogenic bacteria.

In one embodiment, the present invention provides a probiotic preparation comprising the *B. animalis* strain described above.

Preferably, the probiotic preparation is a live bacteria preparation comprising $2 \sim 5 \times 10^{11}$ CFU/g of live bacteria.

Preferably, the probiotic preparation is a dead bacteria preparation comprising $2 \sim 4 \times 10^{11}$ CFU/g of dead bacteria.

In one embodiment, the present invention provides a food or health care product comprising the probiotic preparation described above.

In one example, the food is selected from one or more of a group consisting of milk powder, solid beverage, fermented dairy product, dairy-containing beverage, and cheese.

In one example, the fermented dairy product is selected from a group consisting of fermented bovine milk product and fermented oat milk product.

In another aspect, the present invention provides a method for preparing the *B. animalis* strain described above, comprising: proliferating an appropriate amount of sample in a medium, then treating it with artificial gastric fluid, and artificial intestinal fluid, and isolating the sample to obtain the *B. animalis* strain.

In yet another aspect, the present invention provides use of the *B. animalis* strain described above in the preparation of a medicament for defecation.

The present invention also provides use of the *B. animalis* strain described above in the production of extracellular polysaccharides, lactic acid and short-chain fatty acids.

The present invention also provides use of the *B. animalis* strain described above in the preparation of a medicament for improving immunity and maintaining immune balance.

Preferably, the *B. animalis* strain is used to produce cytokine, which is tumor necrosis factor-$\alpha$ (TNF-$\alpha$) and interleukin-6(IL-6).

Preferably, the single-strain powder of *B. animalis* is used to enhance the mouse cellular immune function and monocyte-macrophage function.

In another aspect, the present invention also provides a lyophilized preparation of *B. animalis* strain, comprising the *B. animalis* strain described above and a lyoprotectant.

In one example, the lyoprotectant comprises: 100-150 g/L of skim milk powder, 20-50 g/L of sucrose, 1-2 g/L of vitamin C, and 0.5-1 g/L of sodium L-glutamate.

In yet another aspect, the present invention provides use of the lyophilized preparation of *B. animalis* strain as described above in the preparation of a medicament for defecation.

The present invention also provides use of the lyophilized preparation of *B. animalis* strain for producing extracellular polysaccharides, lactic acid and short-chain fatty acids.

The present invention also provides use of the lyophilized preparation of *B. animalis* strain as described above in the preparation of a medicament for improving immunity and maintaining immune balance; optionally, the lyophilized preparation is used to produce cytokine, which is tumor necrosis factor-$\alpha$ (TNF-$\alpha$) and interleukin-6 (IL-6).

In yet another aspect, the present invention provides a method for preparing the lyophilized preparation of the *B. animalis* strain as described above, comprising the following steps:

strain culture: inoculating the *B. animalis* strain into a sterile liquid medium at an inoculation amount of 1-3% of the total amount of the medium, culturing for 16-24 hours to obtain a seed culture solution, then inoculating the obtained seed culture solution into a fermentation medium at an inoculation amount of 1-3% of the total amount of the medium, and performing fermentation culture to obtain a fermentation broth of the *B. animalis* strain; and

(2) drying the fermentation broth.

In one example, the fermentation medium comprises: 40-60 g/L of glucose, 60-100 g/L of yeast extract, 3-10 g/L of sodium acetate trihydrate, 0.1-0.2 g/L of magnesium sulfate, 0.05-0.1 g/L of manganese sulfate, 1-2 g/L of dipotassium hydrogen phosphate, 2-4 g/L of triammonium citrate, 1-2 g/L of Tween 80, 0.05-0.1 g/L of calcium chloride, and 0.5-1 g/L of L-cysteine salt.

In another example, during the fermentation, the sodium hydroxide solution is automatically added to maintain a constant pH of 5.5-6.5 to cause the fermentation until acid production is stopped, and the fermentation is terminated when the sodium hydroxide is no longer added.

In another example, the preparation is a live bacteria preparation, and the method further comprises preparing a lyoprotectant after the strain culturing step and before the drying step, and freeze-drying the lyoprotectant in the drying step; preferably, the lyoprotectant used comprises: 80-100 g/L of skim milk powder, 40-50 g/L of trehalose, 2-3 g/L of vitamin C, 4-5 g/L of L-sodium glutamate; preferably, the freeze-drying conditions are: the pre-freezing temperature is -40 ~ -45°C, the pre-freezing time is 4 ~ 5 h, the primary drying temperature is -20 ~ -15°C, the primary drying time is 20 ~ 25 h, the secondary drying temperature is 30 ~ 35°C, and the secondary drying time is 6 ~ 10 h.

In another example, the preparation is a dead bacteria preparation, and the drying step comprising: firstly, performing heat inactivation, and then drying through a spray drying tower to obtain the dead bacteria preparation; optionally, the heat inactivation condition is 80 ~ 100°C for 10 ~ 40 min.

Advantageous Effects

The present invention has the following advantages:

1. The *B. animalis* strain and its probiotic preparation (live and dead bacteria preparations) of the present invention can produce high levels of extracellular polysaccharides, lactic acid and short-chain fatty acids; and can improve the ink propulsion rate, thereby contributing to defecation.

2. Both the *B. animalis* strain and its probiotic preparations (live and dead bacteria preparations) of the present invention can stimulate mouse macrophages RAW264.7 to produce cytokines TNF-$\alpha$ and IL-6. The single-strain powder of *B. animalis* can significantly enhance mouse cellular immune function and monocyte-macrophage function, and can significantly enhance the immunity of the body.

3. The drug comprising the lyophilized preparation of *B. animalis* and the food or health care product comprising the live or dead bacteria preparation of the *B. animalis* strain of the present invention have functions of defecation and enhancing immunity of the body.

4. The method for preparing the lyophilized preparation comprising the *B. animalis* strain of the present invention has simple production process parameters, is easy to control, and has short cycle, that ensures high survival rate of the *B. animalis* strain. The obtained product can be stored for a long time, and the product quality is stable.

## BRIEF DESCRIPTION OF THE DRAWINGS

FIG. 1 shows a phylogenetic tree of *B. animalis* subsp. *lactis* HOM2120 strain.

FIG. 2 shows the RAPD cluster analysis diagram of *B. animalis* subsp. *lactis* HOM2120 strain of the present invention constructed based on the UPGMA method.

FIG. 3 shows the effect of *B. animalis* subsp. *lactis* HOM2120 strain of the present invention on the TNF-$\alpha$ secretion of mouse macrophages.

FIG. 4 shows the effect of *B. animalis* subsp. *lactis* HOM2120 strain of the present invention on the IL-6 secretion of mouse macrophage.

FIG. 5 shows the enhancement of cellular immune function by *B. animalis* subsp. *lactis* HOM2120 of the present invention.

FIG. 6 shows the enhancement of monocyte-macrophage function by *B. animalis* subsp. *lactis* HOM2120 of the present invention.

FIG. 7 shows the function of the single-strain powder of *B. animalis* subsp. *Lactis* HOM2120 of the present invention in improving the ink propulsion rate.

FIG. 8 shows the results of *B. animalis* subsp. *lactis* HOM2120 of the present invention enhancing the mouse cellular immune function, humoral immune function, and monocyte-macrophage function.

## Microbiological Collection

The *Bifidobacterium animalis* (*B. animalis*) HOM2120 strain of the present invention was deposited in the China General Microbiological Culture Collection Center (CGMCC) on September 9, 2022, with a deposit address is: Institute of Microbiology, Chinese Academy of Sciences, No. 3, Courtyard 1, Beichen West Road, Chaoyang District, Beijing; and a deposit number of CGMCC No. 25681.

The *B. animalis* HOM2120 strain of the present invention was sent to the Institute of Microbiology, Chinese Academy of Sciences for identification in May 2023.

The detection and identification conclusions are as follows: Under the conditions of this laboratory, according to the comprehensive analysis of experimental data such as cell morphology, physiological and biochemical characteristics, 16S rRNA gene sequence, *tuf* gene sequence of the test strain, referring to the *"Bergey's Manual of Systematic Bacteriology"* and relevant research paper of International Journal of Systematic and Evolutionary Microbiology, the identification result of the test strain (strain number: HOM2120) is: *Bifidobacterium animalis* subsp. *lactis.*

The cell morphology of this strain is: polymorphic rod-shaped; the physiological and biochemical characteristics are: Gram-positive, catalase negative (-), oxidase negative (-); 16S rRNA gene sequence is shown in SEQ ID NO: 1, and *tuf* gene sequence is shown in SEQ ID NO: 9.

## DETAILED DESCRIPTION

The present invention discloses strains, characteristics and applications, and those skilled in the art can appropriately improve process parameters with reference to the content herein. It should be particularly noted that all similar substitutions and amendments are obvious to those skilled in the art, and are considered to be included in the present invention. The method and application of the present invention have been described through the preferred embodiments. It is obvious that those skilled in the art can make amendments or appropriate changes and combinations to the method and application described herein without departing from the content, spirit and scope of the present invention to implement and apply the technology of the present invention.

In order to further illustrate the technical means and effects adopted by the present invention to achieve the intended purpose, the technical solution of the present invention will be further described below in conjunction with specific examples, but are not limited thereto. Any amendments or equivalent substitutions to the technical solutions of the present invention, without departing from the spirit and scope of the technical solutions of the present invention, shall fall within the protection scope of the present invention. The experimental methods without specific conditions in the following embodiments are implemented according to conventional methods and conditions in the art.

Tumor necrosis factor-$\alpha$ (TNF-$\alpha$) is mainly produced by activated macrophages, NK cells and T lymphocytes, and can regulate the body's immune response. When the immunity of the body is too low, it can activate various immune cells such as lymphocytes and macrophages, enhance their killing activity, and have anti-infection and anti-tumor effects.

Interleukin-6 (IL-6) is mainly produced by monocyte-macrophages, Th2 cells, vascular endothelial cells, and fibroblasts, and can regulate the body's immune response. IL-6 enables the B cell precursor to become an antibody-producing cell; and in synergy with colony stimulating factors, which can promote the growth and differentiation of primitive bone marrow-derived cells and enhances the lytic function of natural killer cells.

As identified above, the *Bifidobacterium animalis* HOM2120 strain in the present invention is identified as *Bifidobacterium animalis* subsp. *lactis.* Therefore, the *Bifidobacterium animalis* HOM2120 strain used in the present invention refers to the *Bifidobacterium animalis* subsp. *lactis* HOM2120 strain.

In order to make the technical problems to be solved of the present invention, technical solutions adopted and advantages clearer, the present invention will be described in detail below with reference to the drawings and specific embodiments. The following examples are intended to illustrate the present invention, but are not intended to limit the scope of the present invention.

It should be noted that the experimental methods used in the present invention are conventional methods unless otherwise specified.

Unless otherwise specified, the reagents and materials used in the present invention are all prepared by conventional methods or obtained commercially.

The *B. animalis* subsp. *lactis* used in the present invention was isolated from feces of healthy infants, and the specific isolation method is as described in Example 1.

**Example**

**Example 1. Isolation and Identification of *B. animalis* subsp. *lactis* HOM2120**

(1) Preparation of Artificial Gastrointestinal Fluid

Artificial gastric fluid: Into 16.4 mL of dilute hydrochloric acid (1 mol/L), 800 mL of water was added, the pH was adjusted to 3.0, and 10g of pepsin was added. The mixture was shaken well, added with water to 1000 mL, centrifuged at 5000 rpm for 5 min, the supernatant was taken, filtered with a 0.22$\mu$m filter membrane for sterilization, and stored at -20°C for later use.

Artificial intestinal fluid: 6.8g of potassium dihydrogen phosphate was weighted, 500 mL of water was added, the pH was adjusted to 6.8 with a 0.4% (0.1 mol/L) sodium hydroxide solution. Separately, 10 g of pancreatin and 3 g of pig bile salt were weighted, an appropriate amount of water was added to dissolve them. The two solutions were mixed, added with water to 1000 mL, centrifuged at 5000 rpm for 5 min. The supernatant was taken, filtered and sterilized with a 0.22 $\mu$m filter membrane, and stored at -20°C for later use.

(2) Preparation of Isolation Medium

MRS-Cys liquid medium: 0.5 g of L-cysteine hydrochloride was added to each liter of MRS broth medium (Cat. No. CM1163, OXOID, UK), sterilized at 121°C for 15 min, and stored at 2 ~ 8°C for one week in the dark.

MRS-Cys solid medium containing bromocresol purple: 0.04g of bromocresol purple was added to each liter of MRS solid medium (Cat. No.: CM1175, OXOID, UK), and stirred well. Sterilization was performed at 121°C for 15 min, about 15 mL of the medium was poured into each petri dish in a clean bench and was allowed to solidify for later use.

(3) Isolation and Screening of *B. animalis* subsp. *lactis* HOM2120

The *B. animalis* subsp. *lactis* of the present invention was isolated from feces of healthy infants.

The extracorporeal feces were collected with a sterile anaerobic tube and stored at low temperature under anaerobic environment. The experiment began on the day of sampling. About 1 g of fecal sample was weighted and added into an anaerobic tube containing 9 mL of MRS-Cys liquid medium, anaerobically incubated at 37°C for 24 h. After centrifugation at 8000 rpm for 10 min, the supernatant was discarded, and 10 mL of artificial gastric fluid was added. After mixing, the mixture was incubated anaerobically at 37°C for 3 h, centrifuged at 8000 rpm for 10 min, the supernatant was discarded. 10 mL of artificial intestinal fluid was added, after mixing, the mixture was incubated anaerobically at 37°C for 3 h. The sample was diluted by a 10-fold dilution method, diluted to a concentration of $10^{-6}$. 100$\mu$L of each dilution was taken from the original solution to the concentration of $10^{-6}$, and spread onto MRS-Cys solid medium plates containing bromocresol purple, anaerobically static incubated at 37°C for 72 h.

Single colonies with yellowing edges were picked, streaked, and purified 3-4 times until single colonies were observed. Gram staining and microscopy of colony morphology were performed simultaneously. Single colonies were then transferred to liquid medium for pure culture, preserved with glycerol, and stored at -80° C.

(4) Morphological Observation of *B. animalis* subsp. *lactis* HOM2120 Strain

*B. animalis* subsp. *lactis* HOM2120 was anaerobically cultured on MRS agar medium at 37°C for 48 h. The colonies were round, milky white, the surface was moist and smooth, the center was raised, and the edges were neat. The diameter of the colonies was about 2.5 mm. It was observed under an optical microscope that the bacteria were rod-shaped with enlarged ends, club-shaped or Y-shaped bacteria. The size of the bacteria was about $(0.5 \sim 0.9)\,\mu m \times (3 \sim 6)$ $\mu m$, arranged individually or in pairs, Gram-positive, and did not form spores.

(5) Identification of *B. animalis* subsp. *lactis* HOM2120 Strain

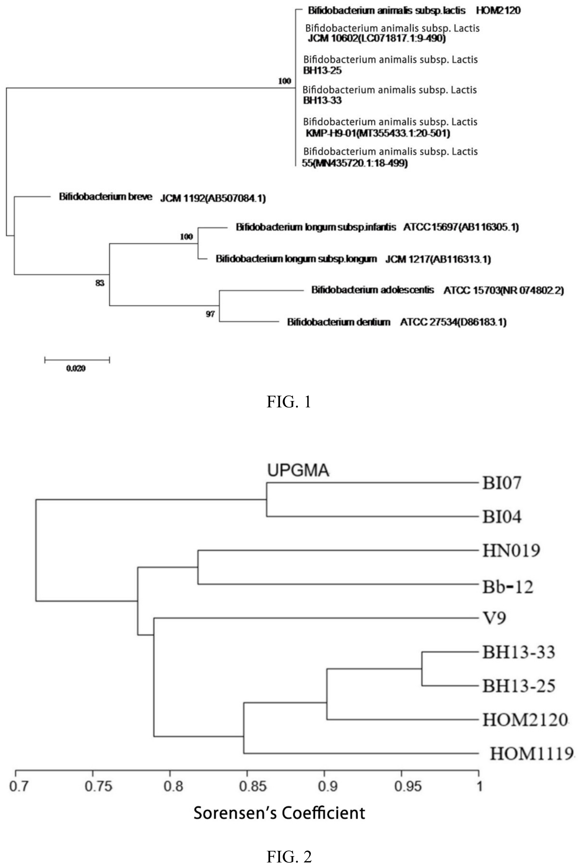

16S rRNA gene identification of the strain: DNA was extracted from the stored strain, and 16S rRNA gene amplification was performed using universal primers 27F: 5'- AGTTTGATCMTGGCTCAG -3'; 1492R: 5'- GGTTACCT TGTTACGACTT -3' (see Table 1) for PCR amplification and agarose gel electrophoresis. Then, the gel was cut and recovered, sequenced, and the isolated strain for 16S rRNA gene sequencing. According to its 16S rRNA gene sequence, it was aligned in the NCBI database using the BLAST tool, and the phylogenetic tree was constructed using Mega 7.0 software, as shown in FIG. 1. The identification result was *B. animalis subsp.lactis,* named HOM2120. The sequence of the 16S rRNA gene is shown in SEQ ID NO: 1:

TGCAGTCGAACGGGATCCCTGGCAGCTTGCTGTCGGGGTGAG

AGTGGCGAACGGGTGAGTAATGCGTGACCAACCTGCCCTGTGCACCGGAA

TAGCTCCTGGAAACGGGTGGTAATACCGGATGCTCCGCTCCATCGCATGGT

GGGGTGGGAAATGCTTTTGCGGCATGGGATGGGGTCGCGTCCTATCAGCTT

GTTGGCGGGGTGATGGCCCACCAAGGCGTTGACGGGTAGCCGGCCTGAGA

GGGTGACCGGCCACATTGGGACTGAGATACGGCCCAGACTCCTACGGGAG

GCAGCAGTGGGGAATATTGCACAATGGGCGCAAGCCTGATGCAGCGACGC

CGCGTGCGGGATGGAGGCCTTCGGGTTGTAAACCGCTTTTGTTCAAGGGCA

AGGCACGGTTTCGGCCGTGTTGAGTGGATTGTTCGAATAAGCACCGGCTAA

CTACGTGCCAGCAGCCGCGGTAATACGTAGGGTGCGAGCGTTATCCGGATT

TATTGGGCGTAAAGGGCTCGTAGGCGGTTCGTCGCGTCCGGTGTGAAAGTC

CATCGCCTAACGGTGGATCTGCGCCGGGTACGGGCGGGCTGGAGTGCGGT

AGGGGAGACTGGAATTCCCGGTGTAACGGTGGAATGTGTAGATATCGGGAA

GAACACCAATGGCGAAGGCAGGTCTCTGGGCCGTCACTGACGCTGAGGAG

CGAAAGCGTGGGGAGCGAACAGGATTAGATACCCTGGTAGTCCACGCCGT

AAACGGTGGATGCTGGATGTGGGGCCCTTTCCACGGGTCCCGTGTCGGAG

CCAACGCGTTAAGCATCCCGCCTGGGGAGTACGGCCGCAAGGCTAAAACT

CAAAGAAATTGACGGGGGCCCGCACAAGCGGCGGAGCATGCGGATTAATT

CGATGCAACGCGAAGAACCTTACCTGGGCTTGACATGTGCCGGATCGCCGT

GGAGACACGGTTTCCCTTCGGGGCCGGTTCACAGGTGGTGCATGGTCGTC

GTCAGCTCGTGTCGTGAGATGTTGGGTTAAGTCCCGCAACGAGCGCAACC

CTCGCCGCATGTTGCCAGCGGGTGATGCCGGGAACTCATGTGGGACCGCC

GGGGTCAACTCGGAGGAAGGTGGGGATGACGTCAGATCATCATGCCCCTTA

CGTCCAGGGCTTCACGCATGCTACAATGGCCGGTACAACGCGGTGCGACA

CGGTGACGTGGGGCGGATCGCTGAAAACCGGTCTCAGTTCGGATCGCAGT

CTGCAACTCGACTGCGTGAAGGCGGAGTCGCTAGTAATCGCGGATCAGCA

ACGCCGCGGTGAATGCGTTCCCGGGCCTTGTACACACCGCCCGTCAAGTCA

TGAAAGTGGGTAGCACCCGAAGCCGGTGGCCCGACCCTTGTGGGGGGA

(6) Identification of strain by random amplification polymorphism DNA (RAPD): DNA was extracted from the stored strain, using the DNA of the strain as a template, and 5 primers OPA-02: TGC CGA GCT G; OPA-18: AGG TGA CCG T; OPL-07: AGG CGG GAA C; OPL-16: AGG TTG CAG G; OPM-05: GGG AAC GTG T were used to amplify DNA fragments capable of showing polymorphism by PCR, different DNA differences were present after gel electrophoresis, and the results were analyzed by cluster analysis software, as shown in FIG. 2. As shown in FIG. 2, *B. animalis* subsp. *lactis* HOM2120 was different from some commercial *B. animalis* subsp. *Lactis* strain and was unique.

Table 1

| Primers | Sequence | SEQ ID NO |
| --- | --- | --- |
| OPA-02 | 5'- TGCCGAGCTG -3' | 2 |
| OPA-18 | 5'- AGGTGACCGT -3' | 3 |
| OPL-07 | 5'- AGGCGGGAAC -3' | 4 |
| OPL-16 | 5'- AGGTTGCAGG -3' | 5 |
| OPM-05 | 5'- GGGAACGTGT -3' | 6 |
| 27F | 5'- AGTTTGATCMTGGCTCAG -3' | 7 |
| 1492R | 5'- GGTTACCTTGTTACGACTT -3' | 8 |

(7) Genome resequencing: DNA was extracted from the stored strain and randomly fragmented. The DNA fragments of a required length were recovered by electrophoresis, and adapters were added for cluster preparation. Sequencing was performed using the Illumina platform. Through data analysis, assembly, and determination, the GC content was found to be 60.44%. By comparing genomics methods, the whole genome obtained by *B. animalis* subsp. *lactis* was re-sequenced and compared with the reference genome of *B. animalis* subsp. *lactis* published on the NCBI website for single nucleotide polymorphism (SNP) analysis. The results were shown in Table 2.

Table 2. SNP analysis results of *B. animalis* subsp. *lactis* HOM2120

| Strains | SNP Differences |
| --- | --- |
| *B. animalis* subsp. *lactis* AD011 | 188 |
| *B. animalis* subsp. *lactis* Bi-07 | 37 |
| *B. animalis* subsp. *lactis* HOM1119 | 74 |
| *B. animalis* subsp. *lactis* DSM.10140 | 35 |
| *B. animalis* subsp. *lactis* HN019 | 40 |
| *B. animalis* subsp. *animalis* IM386 | 60803 |
| *B. animalis* subsp. *lactis* V9 | 34 |

(continued)

| Strains | SNP Differences |
|---|---|
| *B. animalis* subsp. *lactis* BH13-33 | 190 |
| *B. animalis* subsp. *lactis* BH13-25 | 129 |

It can be seen from Table 2 that the SNP differences between *B. animalis* subsp. *lactis* HOM2120 and the reference genome of other *B. animalis* subsp. *lactis* were all not less than 34. The results showed that *B. animalis* subsp. *lactis* HOM2120 was well distinguished from other strains of *B. animalis* subsp. *lactis* and was unique.

**Example 2. Test on the Ability to Inhibit Common Pathogenic Bacteria**

(1) Indicator Bacteria Activation

The indicator strains *Escherichia coli* ATCC8739; *Staphylococcus aureus* ATCC6538; *Salmonella typhimurium* ATCC14028; *Pseudomonas aeruginosa* ATCC9027; *Listeria monocytogenes* ATCC19111 and *Clostridium difficile* ATCC9689 were all purchased from the China Center of Industrial Culture Collection. Indicator strains (*Escherichia coli* ATCC8739; *Staphylococcus aureus* ATCC6538; *Salmonella typhimurium* ATCC14028; *Pseudomonas aeruginosa* ATCC9027; *Listeria monocytogenes* ATCC19111) were inoculated into TSB medium at an inoculation amount of 1% of the total medium, and cultured aerobic at 37°C and 180 rpm for 24h for later use. *Clostridium difficile* ATCC9689 was inoculated into BHI medium at an inoculation amount of 1% of the total amount of the medium, and cultured anaerobically at 37°C for 24 h for later use.

(2) Activation of *B. animalis* subsp. *lactis*

The *B. animalis* subsp. *lactis* HOM2120, cryopreserved at -80°C as prepared in Example 1, and the control strain were respectively inoculated into the sterilized MRS-Cys liquid medium described in Example 1 at an inoculation amount of 1% of the total amount of the medium. After static anaerobic culture at 37°C for 24 hours and activated twice, the strain fermentation broth was obtained. Then, the strain fermentation broth was centrifuged at 8000 rpm for 10 min, and the supernatant was taken for bacteriostatic test.

(3) Plate Preparation

The sterilized TSA medium was heated to completely melt, poured into a petri dish, placed on a horizontal platform to form an agar layer with a uniform thickness, and solidified. The indicator strains were added to the TSA medium, shook evenly, poured into a pre-prepared TSA blank agar plate, and allowed to stand for coagulation.

(4) Bacteriostatic Test

The Oxford cup was gently placed on the plate with sterile forceps, with a certain distance between the holes, and 0.2 mL of the supernatant of the lactic acid bacteria fermentation broth to be tested was respectively added thereto. After the mixture was placed in a refrigerator at 4°C for 24 hours, cultured in an incubator at 37°C for at least 18 hours to observe the occurrence of the inhibition zone. The inhibition zone was formed and then measured with a ruler. The liquid medium (MRS-Cys) in Example 1 was used as a negative control. Each sample was tested in triplicate. The antibacterial results were shown in Table 3. Table 3.

Table 3. Inhibitory Effect of *B. animalis* subsp. *lactis* HOM2120 on Pathogenic Bacteria

| Sample | *E. coli* | *Salmonella* | *Staphylococcus aureus* | *Pseudomonas aeruginosa* | *Listeria monocytogenes* | *Clostridium difficile* |
|---|---|---|---|---|---|---|
| MRS-Cys (Negative Control) | - | - | - | - | - | - |
| BH13-25 | + | + | - | - | + | - |
| BH13-33 | + | + | - | - | + | - |

(continued)

| Sample | E. coli | Salmonella | Staphylococcus aureus | Pseudomonas aeruginosa | Listeria monocytogenes | Clostridium difficile |
|---|---|---|---|---|---|---|
| HOM2120 | + | ++ | +++ | ++ | + | + |

Note: "-" no bacteriostatic activity; "+" 11-16 mm; "++" 17-22 mm; "+++" ≥ 23 mm

It can be seen from Table 3 that *B. animalis* subsp. *lactis* HOM2120 has an inhibitory effect on all 6 pathogenic bacteria. Compared with the other 2 strains of *B. animalis* subsp. *lactis,* the HOM2120 has a better inhibitory effect on *Salmonella, Staphylococcus aureus, Pseudomonas aeruginosa* and *Clostridium difficile*, indicating that it has a better ability to inhibit pathogenic bacteria.

Example 3. Gastrointestinal transit capacity Test

(1) Activation of Strains

The *B. animalis* strain HOM2120, cryopreserved at -80°C as prepared in Example 1, and the control strain were respectively inoculated into the sterilized MRS-Cys liquid medium described in Example 1 at an inoculation amount of 1% of the total amount of the medium, static anaerobic cultured at 37°C for 24 hours. After being activated twice, the strain fermentation broth was obtained.

(2) Preparation of Artificial Gastric Fluid

Into 16.4 ml of dilute hydrochloric acid, about 800 ml of water and 10g of pepsin were added, shaken well, the pH was adjusted to 3.0, the water was added to 1000 ml, and filtered with a 0.2$\mu$m microporous membrane for later use.

(3) Preparation of Artificial Intestinal Fluid

6.8g of potassium dihydrogen phosphate was weighted and dissolved in 500 mL of water, and the pH was adjusted to 6.8. Separately, 10 g of trypsin and 3 g of pig bile salt were weighted, dissolved in an appropriate amount of water. The two solutions were mixed, water was added to 1000 mL, and filtered with a 0.22 um sterile membrane under a sterile environment for later use.

(4) Evaluation of Survival Capacity of Strains in Simulated Gastrointestinal tract

1 mL of activated bacterial solution of the strain to be tested was added to 9 mL of artificial gastric fluid (pH3.0), mixed well, and the number of viable bacteria was counted, and incubated at 37°C for 3 h in an incubator before counting the viable cells again. After culturing in artificial gastric fluid for 3 h, all the bacterial cells were transferred into an equal volume of artificial intestinal fluid (pH6.8), mixed well, and incubated at 37° C. The viable cell counts were performed on MRS medium at 3 h and 24 h respectively, and the survival rate was calculated by the following formula:

Survival rate in the gastric fluid for 3 hours (%) = [logCFUN1/logCFUN0] $\times$ 100%

Survival rate in the intestinal fluid for 3 hours (%)= [logCFUN2/logCFUN0] $\times$ 100%

Survival rate in the intestinal fluid for 24 hours (%) = [logCFUN3/logCFUN0] $\times$ 100%

N0= the viable count of *B. animalis* subsp. *lactis* before treatment, N1= the viable count of *B. animalis* subsp. *lactis* after 3 hours of treatment with gastric fluid, N2= the viable count of *B. animalis* subsp. *lactis* after 3 hours of treatment with intestinal fluid, N3= the viable count of *B. animalis* subsp. *lactis* after 24 hours of treatment with intestinal fluid.
The survival rate of *B. animalis* subsp. *lactis* HOM2120 in the simulated gastrointestinal fluids were shown in Table 4.

Table 4

| Strain Name | Survival rate in gastric fluid for 3 hours | Survival rate in intestinal fluid for 3 hours | Survival rate in intestinal fluid for 24 hours |
|---|---|---|---|
| BH13-33 | 100.27+0.92% | 46.33±4.73%[C] | 33.29±2.59%[C] |
| BH13-25 | 101.04±1.01% | 65.86±1.15%[B] | 42.06±1.99%[B] |
| HOM2120 | 99.13±1.17% | 98.57±1.42%[A] | 91.68±0.47%[A] |

Note: In the same column, different capital letters for the superscript indicate highly significant differences (p < 0.01).

It can be seen from Table 4 that after 3 hours of treatment with simulated gastric fluid, the survival rate of *B. animalis* subsp. *lactis* HOM2120 was higher than 98%. After further treatment with simulated intestinal fluid for 24 hours, the survival rate can still reach up to 91% or more, and was very significantly (p < 0.01) superior to the tolerance to intestinal fluid of the other two strains of *B. animalis* subsp. *lactis*. It indicated that *B. animalis* subsp. *lactis* HOM2120 had a high survival rate in the intestinal tract, laying a foundation for its colonization and effective function in the intestinal tract.

**Example 4. Intestinal Epithelial Cells Adhesion Capacity Test**

(1) Preparation of Cell Medium

Complete medium: Into high-sugar DMEM medium, 10% of inactivated fetal bovine serum (FBS) and 1% (v/v) of antibiotic (100 U/mL penicillin, 100 $\mu$g/mL streptomycin) were added, mixed and then stored at 4° C.
Incomplete medium: Into high-sugar DMEM medium, 10% of inactivated fetal bovine serum (FBS) was added, mixed and then stored at 4° C.

(2) Cell Recovery and Culture

Human colorectal adenocarcinoma Caco-2 cells were purchased from the Cell Resource Center, Institute of Basic Medical Sciences, Chinese Academy of Medical Sciences. The cells were resuspended in a fresh culture solution, uniformly dispersed in a culture flask, cultured at 37°C under a gas condition of 5% $CO_2$ and 95% air. The culture solution was replaced every 48 h during recovery. When the cells grew well (80% confluence), Caco-2 cells were digested with trypsin-EDTA solution at 37°C. The cell concentration was adjusted to $2 \times 10^5$ cells/mL after digestion, and the cells were inoculated in a 24-well plate and cultured until the cells reached 80% confluence.

(3) Activation of *B. animalis* subsp. *Lactis*

The *B. animalis* subsp. *Lactis* HOM2120 cryopreserved at -80°C as prepared in Example 1, was inoculated into the sterilized MRS-Cys liquid medium described in Example 1 at an inoculation amount of 1% of the total amount of the medium, static anaerobic cultured at 37°C for 24 hours and activated twice, and then the strain fermentation broth was obtained.

(4) Adhesion Experiment

The bacterial cells grown in the corresponding medium were collected by centrifugation at 8000 rpm for 10 min. After washing the bacterial cells with DPBS for 3 times, the bacterial cells were resuspended in the incomplete medium, and the concentration of the bacterial cells was adjusted to $10^8$ cfu/mL. 1 mL of the above bacterial suspension was added to a 24-well plate containing Caco-2 cells that have grown to a single layer, and incubated at 37°C for 2 h in a 5% $CO_2$ incubator. After incubation, the cells were washed with sterile DPBS for 3 times. The Caco-2 cells were digested with trypsin-EDTA solution at a temperature of 37°C, the number of cells and the number of viable bacteria before and after adhesion were counted. Each sample was tested in triplicate, and the results were shown in Table 5.

Table 5. Adhesion Capacity of *B. animalis* subsp. *lactis* HOM2120 to Caco-2 cells

| Strain Name | Adhesion Rate (%) | Adhesion Index (CFU/Cell) |
|---|---|---|
| BH13-33 | 1.97±0.73%[B] | 1.82±0.77[b] |
| BH13-25 | 2.44±0.56%[B] | 2.48±0.30[ab] |

(continued)

| Strain Name | Adhesion Rate (%) | Adhesion Index (CFU/Cell) |
|---|---|---|
| HOM2120 | 3.78±0.12%[A] | 3.29±0.15[a] |

Note: In the same column, different capital letters for the superscript indicate highly significant differences (p < 0.01), and different lowercase letters indicate significant differences (p < 0.05).

The results showed that the adhesion rate of *B. animalis* subsp. *lactis* HOM2120 to human colon cancer cells Caco-2 was 3.78, and the adhesion index was 3.29, which was better than the other two *B. animalis* subsp. *Lactis,* and had better capacity to adhere to intestinal epithelial cells.

Adhesion index = number of bacteria after adhesion/number of cells per plate

Adhesion rate = number of bacteria after adhesion/number of bacteria before adhesion

**Example 5. Test for Production Capacity of Extracellular Polysaccharide (EPS)**

The *B. animalis* subsp. *lactis* HOM2120, cryopreserved at -80°C as prepared in Example 1, was inoculated into the sterilized MRS-Cys liquid medium described in Example 1 at an inoculation amount of 1% of the total amount of the medium, static anaerobic cultured at 37°C for 24 hours. After being activated twice, the strain fermentation broth was obtained. Then, the strain fermentation broth was centrifuged at 8000 rpm for 10 min, and the supernatant was taken and the extracellular polysaccharide content was detected using phenol-sulfuric acid method. The MRS-Cys liquid medium described in Example 1 was used as a negative control. Each sample was performed in triplicate, and the data was analyzed using one-way ANOVA with SPSS software. The results were shown in Table 6.

Table 6. Extracellular polysaccharide (EPS) production capacity of *B. animalis* subsp. *lactis* HOM2120

| Sample | EPS Production (mg/L) |
|---|---|
| MRS- Cys (negative control) | 190±2[D] |
| BH13-25 | 289±3[C] |
| BH13-33 | 269±5[B] |
| HOM2120 | 497±3[A] |

Note: In the same column, different capital letters for the superscript indicate highly significant differences (p < 0.01).

It can be seen that compared with other two strains of *B. animalis* subsp. *lactis, B. animalis* subsp. *lactis* HOM2120 had a stronger capacity to produce extracellular polysaccharide (EPS).

**Example 6. Lactic Acid and Short Chain Fatty Acid Production Ability Test**

The *B. animalis* subsp. *lactis* HOM2120 cryopreserved at -80°C as prepared in Example 1, was inoculated into the sterilized MRS-Cys liquid medium described in Example 1 at an inoculation amount of 1% of the total amount of the medium, static anaerobic cultured at 37°C for 24 hours. After being activated twice, the strain fermentation broth was obtained. Then the broth was centrifuged at 8000 rpm for 10 min, and the supernatant was taken and the contents of lactic acid and short-chain fatty acids was determined by gas chromatography. The MRS-Cys liquid medium described in Example 1 was used as a negative control, the other two strains of *B. animalis* subsp. *lactis* were used as positive controls. Each sample was performed in triplicate. The data were analyzed by SPSS25.0 software for one-way ANOVA analysis and Duncan's multiple comparison.

Table 7. Lactic acid and short-chain fatty acid production capacity of *B. animalis* subsp. *lactis* HOM2120

| Sample | Lactic Acid (g/L) | Acetic Acid (g/L) | Formic Acid (g/L) | Propionic Acid (mg/L) | Butyric Acid (mg/L) | Isobutyric Acid (mg/L) |
|---|---|---|---|---|---|---|
| MRS-Cys (Negative Control) | 0.54±0.01[C] | 2.13±0.01[C] | 0.16±0.00[B] | 11.4±0.2[B] | 12.3±0.3[D] | 10.0±0.2[C] |
| BH13-25 | 0.93±0.04[B] | 4.07±0.10[B] | 0.19±0.01[B] | 19.7±0.1[B] | 14.4±0.1[C] | 10.4±0.1[BC] |
| BH13-33 | 0.92±0.01[B] | 4.04±0.03[B] | 0.17±0.01[B] | 20.3±0.4[B] | 15.2±0.2[B] | 10.8±0.1[B] |

(continued)

| Sample | Lactic Acid (g/L) | Acetic Acid (g/L) | Formic Acid (g/L) | Propionic Acid (mg/L) | Butyric Acid (mg/L) | Isobutyric Acid (mg/L) |
|---|---|---|---|---|---|---|
| HOM2120 | $1.76 \pm 0.01^A$ | $4.36 \pm 0.09^A$ | $1.05 \pm 0.05^A$ | $21.6 \pm 0.1^A$ | $16.3 \pm 0.3^A$ | $12.0 \pm 0.3^A$ |

Note: In the same column, different capital letters for the superscript indicate highly significant differences (p < 0.01).

It can be seen from Table 7 that compared with other two strains of *B. animalis* subsp. *lactis, B. animalis* subsp. *lactis* HOM2120 had a stronger capacity to produce lactic acid and short-chain fatty acids (acetic acid, formic acid, propionic acid, butyric acid and isobutyric acid).

## Example 7. In Vitro Cytokine Secretion Promoting Test

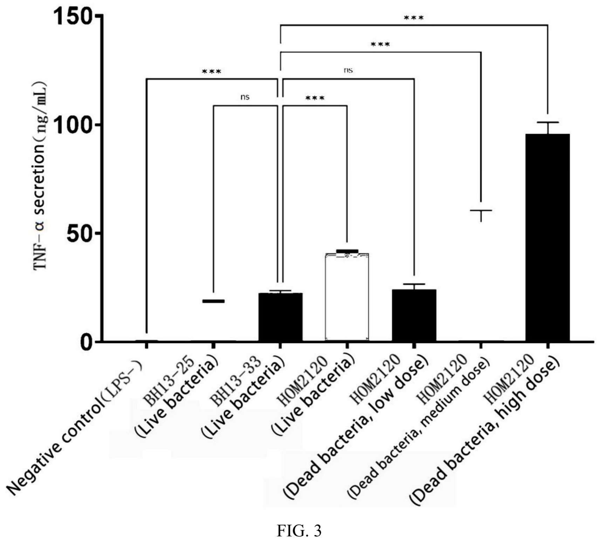

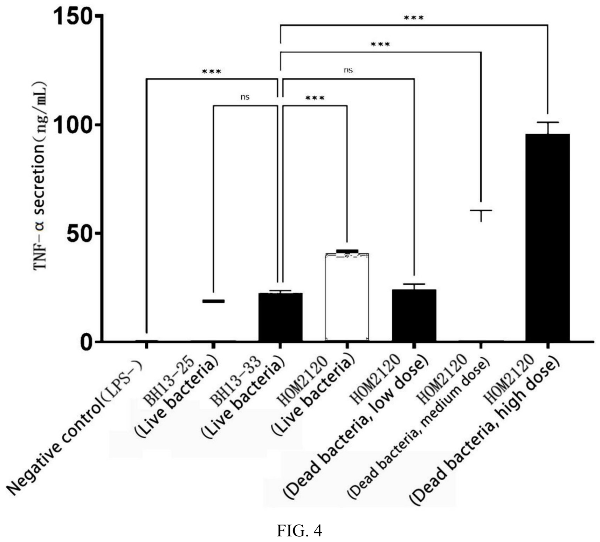

The *B. animalis* subsp. *lactis* HOM2120 cryopreserved at -80°C as prepared in Example 1, was inoculated into the sterilized MRS-Cys liquid medium described in Example 1 at an inoculation amount of 1% of the total amount of the medium, static anaerobic cultured at 37°C for 24 hours. After being activated twice, it was used for viable counting and cell experiments. It was centrifuged at 8000 rpm for 10 min to collect bacterial cells. The DMEM complete medium without antibiotics was used to adjust the concentration of bacterial cells to working level (about $5.0 \times 10^5$ CFU/mL) as a live bacteria group. Moreover, after the bacterial cells were collected, heated at 100°C for 30 min, and then the bacterial count was adjusted to about $5.0 \times 10^5$ CFU/mL as the low-dose group of dead bacteria, to about $5.0 \times 10^6$ CFU/mL as the medium dose group of dead bacteria, and to about $5.0 \times 10^7$ CFU/mL as the high-dose group of dead bacteria. The mouse macrophages RAW264.7 (about $5 \times 10^5$ cell/mL) were added to a 24-well culture plate, with 1 mL per well. After 2 h of adhesion, the medium was discarded, and 1 mL of bacteria-containing medium was added to each well. A blank control group was set, with 1 mL of DMEM medium was added. After 24 h of co-culture, the cell supernatant was collected, and the contents of TNF-$\alpha$ and IL-6 in the cell supernatant were determined by enzyme-linked immunosorbent assay (ELISA) according to the instructions of the kit. The MRS-Cys liquid medium described in Example 1 was used as a negative control, the same dose of viable strains of *B. animalis* subsp. *Lactis* BH13-25 and BH13-33 were used as a positive control. Each sample was prepared in triplicate. Each sample was prepared in triplicate. The data were analyzed by using GraphPad prism9 software for one-way ANOVA and Dennett's t-test (pairwise comparison of means between multiple experimental groups and a control group). The results were shown in FIG. 3 and FIG. 4.
As can be seen from FIG. 3 and FIG. 4, compared with *B. animalis* subsp. *lactis* BH13-25 and BH13-33, both live bacteria and dead bacteria of *B. animalis* subsp. *lactis* HOM2120 can significantly (p < 0.001) improve the secretion of TNF-$\alpha$ and IL-6 by RAW264.7 cells. Under the same dosage condition, live bacteria were superior to dead bacteria; with the increase of the dosage, the immunity was enhanced. It indicated that *B. animalis* subsp. *lactis* HOM2120 had potential immunomodulatory capacity.

## Example 8. Antibiotic Susceptibility Test

The drug susceptibility test was carried out according to ISO10932-2010, "Milk and milk products - Determination of the minimal inhibitory concentration (MIC) of antibiotics applicable to bifidobacteria and non-enterococcal lactic acid bacteria (LAB)", established by the International Organization for Standardization.

(1) Preparation of Medium

MRS-Cys liquid medium: preparation method is the same as in Example 1.
LSM-Cys liquid medium: 21.06g of Iso-Sensitest medium (Cat. No. CM0473B, OXOID, UK) was weighed, 5.2 g of MRS Broth and 0.3 g of L-cysteine hydrochloride were weighed, added with water to 0.5 L, the pH was adjusted to 6.85 $\pm$ 0.1, and sterilized at 121°C for 15 min, and the pH should be 6.7 + 0.1 for later use, and stored at 2 - 8°C for one week in the dark.

(2) Activation and Proliferation of *B. animalis* subsp. *lactis*

The *B. animalis* subsp. *lactis* HOM2120 cryopreserved at -80°C as prepared in Example 1, was inoculated into the sterilized liquid medium (MRS-Cys) described in Example 1 at an inoculation amount of 1% of the total amount of the medium, static anaerobic cultured at 37°C for 24 hours. After being activated twice, the strain fermentation broth was obtained. The activated *B. animalis* subsp. *lactis* HOM2120 was propagated on MRS-Cys agar medium, and static anaerobic cultured at 37°C for 48 hours.

(3) Preparation of Antibiotic Microdilution Plate

0.0512g of antibiotic was weighed, into which 10 mL of solvent was added, wherein chloramphenicol and erythromycin were dissolved in ethanol (without filtration), ampicillin was dissolved in phosphate buffer (pH 8.0, 0.1 mol/L), and other antibiotics were dissolved in water. After shaking to dissolve, filtered with a 0.22μm filter membrane, sub-packaged into EP tubes, with a concentration of 5120 μg/mL (5.12 mg/mL), and stored at -20°C for later use. The antibiotic stock solution was diluted with water (ampicillin with phosphate buffer) to a suitable concentration range. 50μL of diluent was added to the wells of the microdilution plate.

(4) Preparation of Bacterial Suspensions

Individual colonies were picked from agar plates. The obtained colonies were suspended in sterile culture tubes containing 2 mL to 5 mL of sterile saline. The obtained colonies were suspended in pre-reduced LSM-Cys liquid medium. Colonies were suspended until the solution turbidity reached McFarland Standard 1 or the optical density at 625 nm was 0.16 - 0.2 using the spectrophotometer. The suspension was approximately equivalent to $3 \times 10^8$ CFU/mL. The bacterial suspension was diluted with the recommended medium and the bacterial suspension was diluted 500-fold with MRS-Cys liquid medium, as the antibiotic solution will dilute the medium by two times. The diluted bacterial suspension was dispensed within 30 minutes after the preparation. When the freeze plate is used, the frozen antibiotic solution was thawed under anaerobic conditions immediately prior to use. 50μL of the diluted suspension was dispensed into each well of the microdilution plate (about $3 \times 10^4$ CFU/well). The plates were incubated at 37°C under static anaerobic conditions for 48 h. When an anaerobic jar was used, the plates were capped between each plate to create a homogeneous environment in the anaerobic jar. Each experiment was repeated three times, with both positive control group and a negative control group were set. The positive control wells did not contain antibiotics, but included the test strains and medium containing solvents to dissolve the highest concentrations of antibiotics. Negative control wells did not contain the test strain and antibiotics, but included medium.

(5) Reading MIC Results

After 48 hours of incubation, the MIC was visually read. After incubation, the negative control wells were checked for visible bacterial growth. If contamination was found, all data generated by the relevant strain was rejected. Note: If there was no growth in the positive control well, it indicated that the tested strain was sensitive to the solvent used to dissolve the antibiotic. In this case, reading the MIC for that particular antibiotic was meaningless. If the negative and positive controls were normal, the growth of bacteria in each antibiotic was determined visually by comparing them with the positive control. Preferably, the microdilution plate was placed on the top of the frame of the magnifying glass, and the table lamp providing indirect light for easy reading. The growth of bacteria was easily detected under the magnifying glass as sediment at the bottom of the well. Any series of wells in which growth discontinuities were observed were discarded (e.g., grown at 16 μg/mL and 64 μg/mL, but not at 32μg/mL). The endpoint was defined as the lowest antibiotic concentration at which no growth was visually observed. The concentration is the MIC of that antibiotic for that specific strain. Each sample was tested in triplicate, and *Lactobacillus plantarum* ATCC14917 was used as a positive control strain. The antibiotic susceptibility results of *B. animalis* subsp. *lactis* HOM2120 were shown in Table 8. It can be seen from Table 8 that the MIC results of *Lactobacillus plantarum* ATCC14917 were consistent with those shown in the ISO10932-2010 appendix, indicating that the experimental method was accurate. According to the antibiotic resistance standards for bacterial strains used in food established by the European Food Safety Authority (EFSA) in 2012, the antibiotic resistance standard for using strains in food was formulated, and it can be seen that *B. animalis* subsp. *lactis* HOM2120 was sensitive to erythromycin, chloramphenicol, vancomycin, ampicillin, clindamycin, streptomycin, and kanamycin. Therefore, the product of *B. animalis* subsp. *lactis* HOM2120 was relatively safe.

Table 8. Antibiotic susceptibility results of *B. animalis* subsp. *lactis* HOM2120

| Antibiotics | Judgment Criteria | | *B. animalis* subsp. *lactis* HOM2120 | | *Lactobacillus plantarum* ATCC14917 (Control Strain) | |
| --- | --- | --- | --- | --- | --- | --- |
| | Sensitivity (S) | Resistance (R) | MIC Value | Results | MIC Value | Results |
| Erythromycin | ≤1 | >1 | 0.5+0 | S | 1±0 | S |
| Chloramphenicol | ≤4 | >4 | 1.6+0.5 | S | 0.83±0.29 | S |
| Tetracycline | ≤8 | >8 | 16+0 | R | 8±0 | S |

(continued)

| Antibiotics | Judgment Criteria | | B. animalis subsp. lactis HOM2120 | | Lactobacillus plantarum ATCC14917 (Control Strain) | |
|---|---|---|---|---|---|---|
| | Sensitivity (S) | Resistance (R) | MIC Value | Results | MIC Value | Results |
| Vancomycin | ≤2 | >2 | 0.5+0 | S | 128±0 | / |
| Ampicillin | ≤2 | >2 | 0.5+0 | S | 0.5±0 | S |
| Clindamycin | ≤1 | >1 | 0.063+0 | S | 0.5±0 | S |
| Gentamicin | ≤64 | >64 | 107+37 | R | 4±0 | S |
| Streptomycin | ≤128 | >128 | 64±0 | S | 128±0 | / |

**Example 9. Preparation Process 1 of Active Bacterial Powder of *B. animalis* subsp. *lactis* HOM2120**

(1) Culture of Strain

The *B. animalis* subsp. *lactis* HOM2120 cryopreserved at -80°C as prepared in Example 1, was inoculated into the sterilized MRS-Cys liquid medium at an inoculation amount of 1% of the total amount of the medium, and cultured at 37°C for 16-24 hours. After two such subcultures, activated seed culture solution was obtained. The seed culture solution was inoculated into a fermentation medium M416 at an inoculation amount of 1% of the total amount of the medium, and cultured to constant temperature anaerobically at 37°C with a rotation speed of 50 rpm. During the fermentation, a sodium hydroxide solution was automatically added to maintain the pH constant at 5.0. After fermentation for 16-24 hours, it was transferred to another tank to obtain a high-density culture solution of *B. animalis* subsp. *lactis* HOM2120, with a viable count of up to 22.4 billion CFU/mL. The formulation of the fermentation medium M416 was shown in Table 9.

Table 9. Formulation of Fermentation Medium M416

| Medium Components | Content (g/L) |
|---|---|
| Glucose | 50 |
| Yeast Extract | 80 |
| Magnesium Sulfate | 0.4 |
| Manganese Sulfate | 0.2 |
| Dipotassium hydrogen phosphate | 4 |
| Potassium Dihydrogen Phosphate | 4 |
| Tween-80 | 2 |
| Calcium Chloride | 0.1 |
| L-Cysteine Salt | 1 |

(2) Preparation of Lyoprotectant

A protectant containing 100 g/L skim milk powder, 50 g/L sucrose, 2 g/L vitamin C, and 1 g/L L-cysteine salt was prepared by mixing sterile water with raw materials of the protectant.

(3) Freeze-Drying

The fermentation broth of *B. animalis* subsp. *lactis* HOM2120 after culture was centrifuged at 6500 rpm for 15 min at 2-8°C. The supernatant was discarded, the bacterial sludge was collected. The bacterial sludge was washed with 0.9% sterile physiological saline for 1-2 times. The washed bacterial sludge was mixed with the above-mentioned protectant to achieve a bacterial concentration reached $10^{10}$ CFU/mL or more in the mixed bacterial solution. It was then freeze-dried in a freeze dryer, pre-frozen at -40°C for 4 hours, vacuumized, the temperature was raised to -12 °C, and dried for 22 hours in a primary drying; then the temperature was raised to 35°C, and dried for 7 hours in a secondary drying. After freeze-drying, the bacterial cake was pulverized by a fine grinder to obtain the freeze-dried bacterial powder. The viable count of the freeze-dried bacterial powder reached $4.3 \times 10^{11}$ CFU/g.

**Example 10. Preparation Process 2 of Active Bacterial Powder of *B. animalis* subsp. *lactis* HOM2120**

(1) Culture of Strain

The *B. animalis* subsp. *lactis* HOM2120 cryopreserved at -80°C as prepared in Example 1, was inoculated into the sterilized MRS-Cys liquid medium at an inoculation amount of 3% of the total amount of the medium, and cultured at 37°C for 16-24 hours. After two such subcultures, activated seed culture solution was obtained. The seed culture solution was inoculated into a fermentation medium M453 at an inoculation amount of 3% of the total amount of the medium, and cultured to constant temperature anaerobically at 37°C with a rotation speed of 50 rpm. During the fermentation, a sodium hydroxide solution was automatically added to maintain the pH constant at 5.5. After fermentation for 16-24 hours, it was transferred to another tank to obtain a high-density culture solution of *B. animalis* subsp. *lactis* HOM2120, with a viable count of up to 15.3 billion CFU/mL.
The formulation of the fermentation medium M453 was shown in Table 10.

Table 10

| Medium Components | Content (g/L) |
| --- | --- |
| Glucose | 30 |
| Yeast Extract | 70 |
| Magnesium Sulfate | 0.8 |
| Manganese Sulfate | 0.4 |
| Dipotassium hydrogen phosphate | 1 |
| Potassium Dihydrogen Phosphate | 1 |
| Tween-80 | 1 |
| Calcium Chloride | 0.4 |
| L-Cysteine Salt | 0.5 |

(2) Preparation of Lyoprotectant

A protectant containing 150 g/L skim milk powder, 20 g/L sucrose, 1 g/L vitamin C, and 0.5 g/L L-sodium glutamate was prepared by mixing sterile water with raw materials of the protectant.

(3) Freeze-Drying

The fermentation broth of *B. animalis* subsp. *lactis* HOM2120 after culture, was centrifuged at 6500 rpm for 15 min at 2-8°C. The supernatant was discarded, and the bacterial sludge was collected. The bacterial sludge was washed with 0.9% sterile physiological saline for 1-2 times. The washed bacterial sludge was mixed with the above-mentioned protectant to achieve a bacterial concentration of $10^{10}$ CFU/mL or more in the mixed bacterial solution. It was then freeze-dried in a freeze dryer, pre-frozen at -35°C for 5 hours, vacuumized, the temperature was raised to -10°C, and dried for 15 hours in a primary drying; then the temperature was raised to 30°C, and dried for 10 hours in a secondary drying. After freeze-drying, the bacterial cake was pulverized by a fine grinder to obtain the freeze-dried bacterial powder. The viable count of the freeze-dried bacterial powder reached $3.7 \times 10^{11}$ CFU/g.

**Example 11. Preparation Process of Dead Bacterial Powder of *B. animalis* subsp. *lactis* HOM2120**

(1) Culture of Strain

The strain culture was as described in Example 9 or Example 10.

(2) Preparation of Bacterial Powder

The fermentation broth of *B. animalis* subsp. *lactis* HOM2120 obtained by high-density fermentation was treated at 100°C for 10 minutes, 100g of maltodextrin excipient was added per liter of the fermentation broth, and dried by a spray dryer. The number of dead bacteria in the dead bacterial powder was $2.2 \times 10^{11}$ CFU/g when counted under a microscope using a hemocytometer.

**Example 12. Animal Test to Enhanced Immunity**

(1) Experimental animals and grouping: 192 female SPF-grade KM mice (18-20g) bred in Kunming by Beijing Huafukang Biotechnology Co., Ltd were selected. After animal adaptation and observation, they were randomly divided into four batches, with four groups per batch and 12 mice per group. The experimental groups were divided into three dose groups: a low-dose group ($5 \times 10^9$ CFU/Kg BW), a medium-dose group ($2.5 \times 10^{10}$ CFU/Kg BW) and a high-dose group ($5 \times 10^{10}$ CFU/Kg BW). The mice were administered with the active bacterial powder of *B. animalis* subsp. *lactis* HOM2120 prepared in Example 9 by gavage with physiological saline as the solvent, which was administered orally once a day. Various indicators were measured after 32 days of continuous gavage. Mice were administered with a volume of 10 mL/kg BW via gavage, while the control group was administered with a high dose of physiological saline solution containing the same mass of bacterial powder excipients via gavage. Each dose group was given maintenance feed. Experimental group one underwent carbon clearance test; experimental group two underwent organ/body weight ratio measurement and a delayed-type hypersensitivity experiment; experimental group three underwent mouse peritoneal macrophage phagocytosis of chicken red blood cells experiments; and experimental group four underwent a ConA-induced mouse lymphocyte transformation experiments.
(2) Experimental Methods

A. Determination of Organ/Body Weight Ratio

After weighing, the mice were sacrificed by cervical dislocation. The spleen and thymus were taken, fascia was removed, blood stains were blotted from the surface of the organ with filter paper, and then weighed. The spleen/body weight ratio and thymus/body weight ratio were calculated.

B. Experiment of Delayed Type Hypersensitivity (DTH) Experiment (Plantar Thickening Method)

The sheep blood was taken and washed 3 times with physiological saline, and each mouse was intraperitoneally injected with 0.2 mL of 2% (v/v, prepared with physiological saline) sheep red blood cell (SRBC) suspension (2000 r/min, 10 min). Four days after sensitization, the thickness of the left hind paw plantar region was measured. Then, 20 $\mu$L of 20% (v/v, prepared with physiological saline) SRBC suspension was injected subcutaneously at the measurement site, and the thickness of the left hind paw plantar region was measured 24h after injection. The above two measurements were taken three times at the same site, and the average value was used. The difference in plantar thickness before and after attack was used to represent the degree of DTH. The difference of the test sample group was significantly higher than that of the control group, indicating a positive result for this experiment.

C. ConA-Induced Mouse Lymphocyte Transformation Test (MTT Method)

The spleen was aseptically removed and placed in a small petri dish containing an appropriate amount of sterile Hank's solution. The spleen was gently ground with tweezers to prepare a single-cell suspension. The suspension was filtered through a 200-mesh sieve to prepare a cell suspension. It was washed twice with Hank's solution and centrifuged for 5 min each time (1000 r/min). The cells were then suspended in 1m of complete medium, counted by microscopy, and the cell concentration was adjusted to $3 \times 10^6$ cells/mL. The spleen cell suspension was then added to two wells of a 24-well culture plate at 1.0 mL per well. One well contained 75$\mu$L of ConA solution (equivalent to 7.5$\mu$g/mL), and the other well served as a control. The plate was cultured in a carbon dioxide incubator with 5% CO, at 37°C for 72 h. Four hours before the end of the culture, 0.7 mL of supernatant was gently aspirated from each well, and 0.7 mL of RPMI1640 culture solution (without fetal bovine serum) was added. Simultaneously, 50$\mu$L of MTT (5 mg/mL) per well was added, and incubated for 4 h. After the incubation was completed, 1.0 mL of acidic isopropanol was added to each well, and the mixture was pipetted and mixed to completely dissolve the purple crystals. This solution was then moved into a cuvette and measured colorimetrically at 570 nm using a spectrophotometer. The proliferation capacity of lymphocytes was obtained by subtracting the optical density value of the well containing ConA from the optical density value of the well without ConA. The optical density difference of the test sample group was significantly higher than that of the control group, which indicated that the result of the experiment was positive.

D. Antibody - Forming Cell (PFC) Test (Jerne's Modified Slide Method)

The sheep blood was collected and washed 3 times with physiological saline. Each mouse was intraperitoneally injected with 0.2 mL of 2% (v/v, prepared with physiological saline) SRBC suspension. Five days after SRBC immunization, mice were sacrificed by cervical dislocation. The spleen was taken out and placed in a small petri dish containing an appropriate amount of sterile Hank's solution. The spleen was gently ground to prepare a cell suspension. The suspension was centrifuged (1000 r/min) for 5 min, washed twice with Hank's solution, and finally suspended cells in 8.0 mL of Hank's solution. After the agarose was heated and dissolved, it was mixed with an equal amount of 2-fold concentration of Hank's solution, and dispensed into small test tubes, 0.5 mL of each tube. Then, 50μL of 10% (v/v, prepared with SA solution) SRBC suspension and 8μL of spleen cell suspension were added to the tubes, mixed rapidly, poured onto glass slides coated with a thin layer of agarose, parallel slides were prepared. After the agarose solidified, the slides were horizontally placed on a slide holder, placed in a carbon dioxide incubator and incubated at 37°C for 1 h. Then, complement diluted with SA buffer (1 : 8) was added into the grooves of the slide holder, and continued incubation for 1.5 h. The number of hemolytic plaques was counted. It was expressed by the number of plaques/whole splenocytes. The number of plaques in the test sample group was significantly higher than that in the control group, which indicates that the result of the experiment is positive.

E. Determination of Half Hemolysis Value (HC50)

Sheep blood was collected and washed three times with physiological saline. Each mouse was immunized by intraperitoneal injection of 0.2 mL of 2% (v/v, prepared with physiological saline) SRBC suspension. After 5 days, the eyeballs were removed, and blood was collected in a centrifuge tube. After standing for about 1 hour, the clotted blood was peeled off from the tube wall to allow the serum to be fully separated, centrifuged at 3000 r/min for 10 min and the serum was collected. The serum was diluted 300-fold with SA buffer, 1.0 mL of which was taken and placed in a test tube, and 0.5 mL of 10% (v/v, prepared with SA buffer) SRBC suspension and 1.0 mL of complement (diluted with SA buffer at 1 : 8) were added sequentially. A control tube without serum (replaced with SA buffer) was also provided. After heat preservation in a 37°C constant-temperature water bath for 15 min, the reaction was terminated in an ice bath. The control tube was centrifuged at 2000 r/min for 10 min, 1.0 mL of supernatant was collected, and 3.0 mL of Hb diluent was added. At the same time, 0.25 mL of 10% SRBC suspension (v/v, prepared with SA buffer) was taken, added with Hb diluent to a final volume of 4.0 mL in another test tube, mixed well. After standing for 10 minutes, the optical density value of each tube was measured at 540 nm using the control tube as a blank. The amount of hemolysin is expressed as half hemolysis value (HC50), which was calculated according to the following formula: half hemolysis value of sample = sample optical density value / SRBC optical density value at half hemolysis $\times$ dilution factor. The HC50 of the test sample group was significantly higher than the HC50 of the control group, which indicates that the result of the experiment is positive.

F. Mouse Carbon Clearance Test

4-fold diluted Indian ink (0.05 mL/10 gBW) was injected into the tail vein of mice according to body weight. After the ink was injected, timing was performed immediately. After 2 min and 10 min of ink injection, 20μL of blood was taken from the venous plexus of angular vein and added to 2.0 mL of 0.1% $Na_2CO_3$ solution, respectively. A spectrophotometer was used to measure optical density (OD) at a wavelength of 600 nm, and a 0.1% $Na_2CO_3$ solution was used as a blank control. Mice were sacrificed, and livers and spleens were weighed. The phagocytic index (a) represents the mouse's carbon clearance capacity. (a) is calculated as follows:

$$k = (lgOD1 - lgOD2)/(t2 - t1), a = body\ weight \div (liver\ weight + spleen\ weight) \times k^{1/3}$$

The phagocytic index of the test sample group was significantly higher than that of the control group, which indicated that the result of the experiment was positive.

G. Experiment of Mouse Intraperitoneal macrophages phagocytosing Chicken Red Blood Cells (Semi-In-Vivo Method)

Mice were intraperitoneally injected with 1 mL of 20% (v/v, prepared with physiological saline) chicken red blood cell (2000 r/min, 10 min) suspension. After 30 min intervals, the mice were sacrificed by cervical dislocation, and fixed in supine position on a mouse plate. 2 mL of physiological saline was injected intraperitoneally, and the abdomen was gently massaged for 20 times. 1 mL of peritoneal macrophage washing solution was taken and dropped on two glass slides respectively, placed in an enamel box padded with wet gauze, and incubated in an incubator at 37°C for 30 min. After incubation, the slides were rinsed in physiological saline to remove unattached cells. The slides were air-dried, fixed with 1:1 acetone methanol solution, stained with Giemsa-phosphate buffer, rinsed with distilled water, and dried in air. Macrophages were counted under an oil immersion microscope, with 100 macrophages counted per slide. The phagocytic rate and phagocytic index were calculated using the following formulas:

Phagocytic percentage (%) = number of macrophages phagocytosing chicken red blood cells / number of macrophages counted $\times$ 100%

Phagocytic index = Total number of phagocytosed chicken red blood cells / number of macrophages counted

The obtained phagocytic percentage was then converted using the following formula: $X = \mathrm{Sin}^{-1}\sqrt{P}$, where P was the phagocytic percentage, expressed as a decimal. The obtained data were quantitative data. The phagocytic percentage and phagocytic index of the test sample group were significantly higher than those of the control group, which indicated that the result of the experiment was positive.

H. Determination of NK Cell Activity (Lactate Dehydrogenase LDH Assay)

The target cells YAC-1 were subcultured at 24h before the experiment. The cells were washed 3 times with Hank's solution before use, and the cell concentration was adjusted to $4 \times 10^5$ cells/mL with RPMI1640 complete medium (containing 10% fetal bovine serum). The tested mice were sacrificed by cervical dislocation. The spleen was taken out aseptically, and spleen cell suspensions were prepared, washed twice with Hank's solution, and centrifuged for 10 min each time (1000 r/min). The supernatant was discarded and the cell pellet was resuspended, 0.5 mL of sterilized water was added for 20 seconds, red blood cells were lysed, then 0.5 mL of 2-fold Hank's solution and 8.0 mL of Hank's solution were added. It was centrifuged at 1000 r/min for 10 min, resuspended with 1.0 mL of RPMI1640 complete culture solution (containing 10% fetal bovine serum), and counted by microscopic examination, and the cell concentration was adjusted to $2 \times 10^7$ cells/mL with the RPMI1640 complete culture solution. The effector-to-target ratio was at 50 : 1. 100μL of the target cells and 100μL of the effector cells were taken and added to a U-shaped 96-well culture plate. 100μL of the target cells and 100μL of the medium were added to the target cell natural release wells, and 100μL of the target cells and 100μL of the 1% NP40 were added to the target cell maximum release wells. Each of these wells were provided with three parallel wells. The cells were cultured in a carbon dioxide incubator at 37°C and 5% $CO_2$ for 4 h. The 96-well plate was centrifuged at 1500 r/min for 5 min, 100μL of supernatant from each well was pipetted and placed in a flat-bottomed 96-well culture plate, 100μL of LDH matrix solution was added to react for 10 min. Then, 30μL of 1 mol/L HCl solution was added to each well to terminate the reaction. The optical density (OD) was measured at 490 nm using a microplate reader. NK cell activity was calculated: NK cell activity (%) = (OD of reaction well - OD of natural release well) / (OD of maximum release well - OD of natural release well) × 100%

The obtained NK cell activity was converted using the following formula: $X = \mathrm{Sin}^{-1}\sqrt{P}$, where P was the NK cell activity expressed as a decimal. The obtained data were quantitative data. The NK cell activity of the test sample group was significantly higher than that of the control group, which indicated that the result of the experiment was positive.

(3) Data Processing

Data processing was performed with SPSS software. The analysis of variance was adopted, but a homogeneity of variance test was performed first according to the ANOVA procedure. If the variance were homogeneous, the F value was calculated. If the F value is < F0.05, the conclusion was: there was no significant difference between the averages of each group. If the F value is ≥ F0.05, P ≤ 0.05, Duncan's multiple comparison method was used for statistical analysis. For non-normal or unequal variance data, appropriate variable transformations were performed until the data met the requirements of normality or homogeneity of variance. The transformed data were used for statistics analysis. If the transformations still did not achieve normality or homogeneity of variance, the rank-sum test was used for statistical analysis.

(4) Experimental Results

Table 11 shows the differences in body weight before and after the experiment among mice in the high, medium, and low dose groups of *B. animalis* subsp. *lactis* HOM2120 and control group.

Table 11

| Group | n | Initial Body Weight (g) Batch 1 | Batch 2 | Final Body Weight (g) Batch 1 | Batch 2 |
|---|---|---|---|---|---|
| G1 (control group) | 12 | 21.2±1.1 | 21.1+1.0 | 36.2±2.5 | 36.3±2.5 |
| G2 (HOM2120 low-dose group) | 12 | 21.2+0.8 | 21.2±0.7 | 37.1±1.9 | 36.8±2.4 |
| G3 (HOM2120 medium-dose group) | 12 | 21.2+0.6 | 21.2+0.9 | 36.0±2.1 | 36.3±2.6 |
| G4 (HOM2120 high-dose group) | 12 | 21.2±1.0 | 21.2±0.9 | 36.0+2.4 | 36.0±2.7 |

(continued)

| Group | n | Initial Body Weight (g) Batch 3 | Batch 4 | Final Body Weight (g) Batch 3 | Batch 4 |
|---|---|---|---|---|---|
| G1(control group) | 12 | 21.1±1.1 | 21.1+1.1 | 37.0±2.3 | 36.9+2.4 |
| G2 (HOM2120 low-dose group) | 12 | 21.2+0.9 | 21.2±0.6 | 37.9±3.3 | 35.1±2.4 |
| G3 (HOM2120 medium-dose group) | 12 | 21.2±0.8 | 21.2+0.9 | 35.7±3.1 | 36.6±2.6 |
| G4 (HOM2120 high-dose group) | 12 | 21.2+0.7 | 21.2+0.9 | 36.5±2.6 | 36.1±3.5 |

Note: Comparison between each group and the negative control group, p > 0.05

It can be seen from the results in Table 11 that, compared with the control group, there was no significant difference (p > 0.05) in body weight before and after the experiment in the high, medium, and low dose groups of *B. animalis* subsp. *lactis* HOM2120. This indicated that the active bacterial powder of *B. animalis* subsp. *lactis* HOM2120 had no effect on the body weight of the mice.

Table 12 shows the effect of *B. animalis* subsp. *lactis* HOM2120 bacterial powder on the organ/body weight ratio in mice ($\overline{x}\pm S$).

Table 12

| Group | n | Spleen/Body (mg/g) | Thymus/Body (mg/g) |
|---|---|---|---|
| G1(control group) | 12 | 4.67±1.25 | 3.67+0.58 |
| G2 (HOM2120 low-dose group) | 12 | 4.23+0.51 | 3.58+0.75 |
| G3 (HOM2120 medium-dose group) | 12 | 4.46+0.90 | 3.68+0.81 |
| G4 (HOM2120 high-dose group) | 12 | 4.23+0.86 | 3.64+0.60 |

Note: Comparison between the test group and the negative control group, compared, p > 0.05

As can be seen from the results in Table 12, compared with the control group, there was no significant difference (p > 0.05) in spleen/body weight and thymus/body weight of mice in the high, medium and low dose groups of *B. animalis* subsp. *lactis* HOM2120 active bacterial powder. This indicated that the *B. animalis* subsp. *lactis* HOM2120 active bacterial powder had no effect on the spleen and thymus weights of mice.

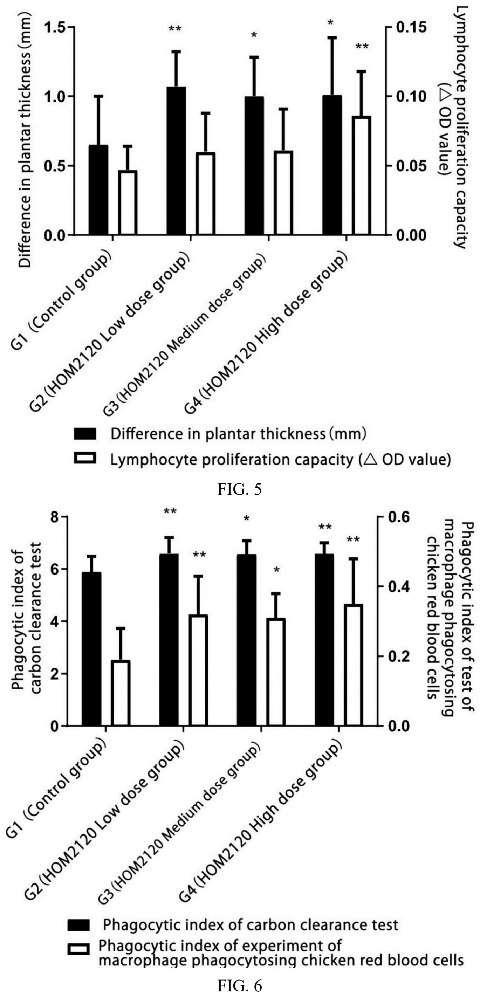

Table 13 and FIG. 5 show the effects of *B. animalis* subsp. *lactis* HOM2120 bacterial powder on mice DTH induced by sheep red blood cells (SRBC) and on proliferation capacity of mouse spleen lymphocytes induced by ConA($\overline{x}\pm S$).

Table 13

| Group | n | Cell Immune Function Plantar Thickness Difference (mm) | Lymphocyte Proliferation Capacity (ΔOD Value) |
|---|---|---|---|
| G1(control group) | 12 | 0.65+0.35 | 0.047+0.017 |
| G2 (HOM2120 low-dose group) | 12 | 1.07±0.25** | 0.060+0.028 |
| G3 (HOM2120 medium-dose group) | 12 | 1.00+0.28* | 0.061±0.030 |
| G4 (HOM2120 high-dose group) | 12 | 1.01+0.41* | 0.086+0.032** |

Note: * indicates p < 0.05 compared to the control group; ** indicates p < 0.01 compared to the control group; *** indicates p < 0.001 compared to the control group.

As can be seen from the results in Table 13 and FIG. 5, compared with the control group, in the *B. animalis* subsp. *lactis* HOM2120 active bacterial powder, the active bacterial powder in the high dose group can significantly improve the degree of mouse paw plantar thickening induced by sheep red blood cell (p < 0.05); and the active bacterial powder in the low dose group can significantly improve the degree of mouse paw plantar thickening induced by sheep red blood cell (p < 0.01). High dose group of *B. animalis* subsp. *lactis* HOM2120 active bacterial powder can significantly improve the proliferation capacity of mouse spleen lymphocytes induced by ConA (p < 0.001). This indicates that *B. animalis* subsp. *lactis* HOM2120 active bacterial powder significantly enhances the cellular immune function of mice.

Table 14 shows the effect of *B. animalis* subsp. *lactis* HOM2120 powder on the number of antibody-producing cells and the half hemolysis value of serum (factor $x\pm S$).

Table 14

| Group | Humoral Immune Function | |
| | Antibody-Producing Cell Experiment The number of hemolytic plaques ($\times 10^3$/whole spleen) | Serum Hemolysin Experiment Half hemolysis value |
|---|---|---|
| G1(control group) | 141±25 | 161±43 |
| G2 (HOM2120 low-dose group) | 140+32 | 164±61 |
| G3 (HOM2120 medium-dose group) | 155±31 | 169+64 |
| G4 (HOM2120 high-dose group) | 161±28 | 178+46 |
| Note: * indicates p < 0.05 compared to G1 group; ** indicates p < 0.01 compared to G1 group; *** indicates p < 0.001 compared to G1 group | | |

It can be seen from the results in Table 14 that, compared with the control group, there were no significant difference in the number of hemolytic plaques of mice and there was no significant difference in the half hemolysis value of mice in serum (p > 0.05) in each dose group of *B. animalis* subsp. *lactis* HOM2120 active bacterial powder (p > 0.05).

Table 15 and FIG. 6 show the effect of *B. animalis* subsp. *lactis* HOM2120 powder on the carbon clearance capacity and on the phagocytic rate and phagocytic index of mouse macrophages phagocytosing chicken red blood cells ($\overline{x}\pm S$).

Table 15

| Group | Monocyte-Macrophage Function | | | |
| | Carbon clearance test | Experiment of macrophages phagocytosing chicken red blood cells | | |
| | Phagocytic index (a) | Phagocytic rate (%) | Square root of phagocytic rate Arcsine transform value | Phagocytic index |
|---|---|---|---|---|
| G1 (control group) | 5.89+0.60 | 12±6 | 0.35+0.10 | 0.19+0.09 |
| G2 (HOM2120 low-dose group) | 6.59+0.61** | 21+8** | 0.47+0.09** | 0.32+0.11** |
| G3 (HOM2120 medium-dose group) | 6.57+0.52* | 20+5* | 0.46+0.06** | 0.31+0.07* |
| G4 (HOM2120 high-dose group) | 6.58+0.42** | 22±7** | 0.48+0.08*** | 0.35±0.13** |

Note: * indicates p < 0.05 compared to G1 group; ** indicates p < 0.01 compared to G1 group; *** indicates p < 0.001 compared to G1 group

It can be seen from the results in Table 15 and FIG. 6 that, compared with the control group, both the low-dose group and the high-dose group of the *B. animalis* subsp. *lactis* HOM2120 active bacterial powder can significantly improve the carbon clearance capacity of mice (p < 0.01); the medium-dose group can significantly improve the carbon clearance capacity of mice (p < 0.05). Both the low-dose group and the high-dose group of active bacterial powder can significantly improve the phagocytic rate (p < 0.01) and phagocytic index (p < 0.01) of mouse macrophages phagocytosing chicken red blood cells; and the medium-dose group can significantly improve the phagocytic rate (p < 0.05) and phagocytic index (p < 0.05) of mouse macrophages phagocytosing chicken red blood cells. This indicated that *B. animalis* subsp. *lactis* HOM2120 active bacterial powder significantly enhanced the function of mouse monocyte-macrophage.

Table 16 shows the effect of *B. animalis* subsp. *lactis* HOM2120 bacterial powder on NK cell activity assay ($\overline{x}\pm S$).

Table 16

| Group | Nk cell activity experiment | |
| | Nk cell activity (%) | Nk cell activity Square root arcsine transformation value |
|---|---|---|
| G1 (control group) | 27.1±5.6 | 0.55±0.06 |
| G2 (HOM2120 low-dose group) | 29.6±4.6 | 0.57±0.05 |
| G3 (HOM2120 medium-dose group) | 30.9±6.7 | 0.59±0.07 |

(continued)

| Group | Nk cell activity experiment | |
|---|---|---|
| | Nk cell activity (%) | Nk cell activity Square root arcsine transformation value |
| G4 (HOM2120 high-dose group) | 30.9±3.5 | 0.59±0.04 |
| Note: Comparison between the test group and the negative control group, p > 0.05 | | |

As can be seen from the results in Table 16, there was no significant difference in NK cell activity of mice in each dose group of *B. animalis* subsp. *lactis* HOM2120 compared with the control group (p > 0.05).

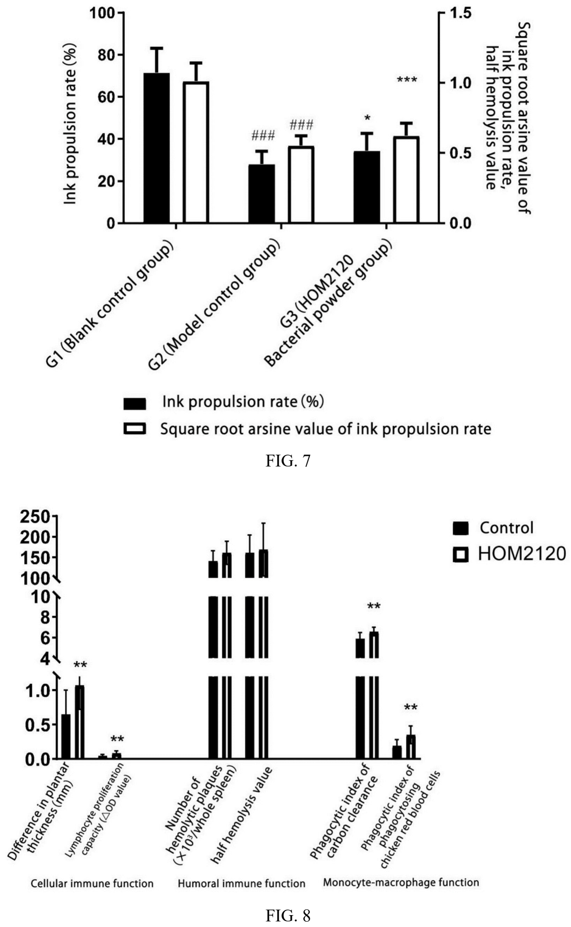

In summary, as shown in FIG. 8, compared with the control group, the *B. animalis* subsp. *lactis* HOM2120 active bacterial powder can significantly enhance the cellular immune function and monocyte-macrophage function of mice, while having no effect on mouse body weight, thymus and spleen weight, humoral immune function and NK cell activity. In summary, the *B. animalis* subsp. *lactis* HOM2120 active bacterial powder has the function of enhancing immunity.

**Example 13. Animal Test of defecation Function**

(1) Experimental animals and grouping: a total of 36 male SPF-grade KM mice (18-20g) bred in Kunming by Beijing Huafukang Biotechnology Co., Ltd were selected. After animal adaptation and observation, they were randomly divided into four groups, with 12 mice in each group for small intestinal animal test. The experimental group was a single-strain powder group *(B. animalis* subsp. *lactis* HOM2120, at a dose of $5 \times 10^{10}$ CFU/Kg BW).

(2) Experimental Methods

The mice were administered via gavage once daily for 15 consecutive days with physiological saline as solvent. Mice in each group were fasted but allowed free access to water for 16 hours. The model control group and the experimental group were administered with compound diphenoxylate (5 mg/KgBW) by gavage, while the blank control group was administered with distilled water by gavage. 0.5 hours after administration of compound diphenoxylate, each sample dosage group was given ink (containing 5% activated carbon and 10% gum arabic) containing the corresponding test sample, and the blank control group and the model control group were administered with ink by gavage. After 25 minutes, the animals were immediately sacrificed by cervical dislocation. The intestinal lumen was opened, the mesentery was separated, and the intestinal segment from the pylorus (upper end) to the ileocecal junction (lower end) was cut. The segment was placed on the tray, and the small intestine was gently pulled into a straight line. The length of the intestinal segment was measured as "the total length of the small intestine", and the length from the pylorus to the front edge of the ink was measured as "ink propulsion length". The ink propulsion rate was calculated using the following formula:

Ink propulsion rate (%) = ink propulsion length (cm) / total length of the small intestine (cm) $\times$ 100%.

(3) Data Processing

Data conversion was required for the ink propulsion rate, $X=\mathrm{Sin}\text{-}1\sqrt{p}$ (p is the ink propulsion rate, expressed as a decimal). SPSS software was used for data processing. Independent sample T-test was used. The statistical results are divided into two parts, i.e., 1: Levene's test for homogeneity of variance; 2: t-test for equality of means. When the result of the homogeneous of variance test is Sig > 0.05, it is considered that the variances are homogeneous, so the Sig value in the first column of the T-test for equality of means is taken. When Sig ≤ 0.05, the statistical results between the two groups are considered to be different. When the result of the homogeneity of variance test is Sig ≤ 0.05, it is considered that the variances are not homogeneous, so the Sig value in the second column of the T-test for equality of means is taken. When Sig ≤ 0.05, it is considered that there is a difference in the statistical results between the two groups.

(4) Experimental Results

Table 17 and FIG. 7 show the effect of *B. animalis* subsp. *lactis* HOM2120 single-strain powder on the ink propulsion rate (x̄±S).

Table 17

| Group | N | Ink propulsion rate (%) | Square root arcsine value of ink propulsion rate |
|---|---|---|---|
| G1 (blank control group) | 12 | 71.4±11.7 | 1.01±0.13 |
| G2 (Model Control) | 12 | 27.9±6.1## | 0.55±0.07## |
| G2 (HOM2120 single-strain powder group) | 12 | 34.2±8.3* | 0.62±0.09* |

Note: # indicates $p < 0.05$ compared to the blank control group; ## indicates $p < 0.01$ compared to the blank control group; ### indicates $p < 0.01$ compared to the blank control group.
* indicates $p < 0.05$ compared to the model control group; ** indicates $p < 0.01$ compared to the model control group; *** indicates $p < 0.001$ compared to the model control group.

It can be seen from the results in Table 17 and FIG. 7 that, after 15 days of oral administration with the test substance *(B. animalis* subsp. *lactis* HOM2120 single-strain powder) to mice, the constipation model of mice was induced using compound diphenoxylate. Compared with the blank control group, the model control group showed a decreased ink propulsion rate, with the P value showed a significant difference, indicating that the model was successfully established. Compared with the model control group, the ink promotion rate of the *B. animalis* subsp. *lactis* HOM2120 single-strain powder group was increased, and the p value showed a significant difference, indicating that the *B. animalis* subsp. *lactis* HOM2120 single-strain powder has the potential of defecation Function.

The above description is only preferred embodiments of the present disclosure, and it should be noted that for those skilled in the art, several improvements and modifications can be made without departing from the principle of the present disclosure, and these improvements and modifications should also be considered within the protection scope of the present invention.

Although the embodiments of the present invention have been disclosed as above, they are not limited to the applications listed in the specification and the embodiments. They can be fully applied to various fields suitable for the present invention, and those skilled in the art can easily realize other modifications. Therefore, without departing from the general concept defined by the claims and the equivalent scope, the present invention is not limited to specific details and the details shown and described herein.

**Claims**

1. A *Bifidobacterium animalis (B. animalis)* strain, wherein the deposit number of the *Bifidobacterium animalis* strain is CGMCC No. 25681.

2. The *B. animalis* strain according to claim 1, wherein the *B. animalis* strain comprises a sequence of 16S rRNA gene represented by SEQ ID NO: 1.

3. A probiotic preparation comprising the *B. animalis* strain according to claim 1.

4. The probiotic preparation according to claim 3, wherein the probiotic preparation is a live bacteria preparation comprising $2 \sim 5 \times 10^{11}$ CFU/g of live bacteria.

5. The probiotic preparation according to claim 3, wherein the probiotic preparation is a dead bacteria preparation comprising $2 \sim 4 \times 10^{11}$ CFU/g of dead bacteria.

6. A food or health care product comprising the probiotic preparation according to any one of claims 3 to 5.

7. The food or health care product according to claim 6, wherein the food is selected from one or more of a group consisting of milk powder, solid beverage, fermented dairy product, dairy-containing beverage and cheese.

8. The food or health care product according to claim 7, wherein the fermented dairy product is selected from a group consisting of fermented bovine milk product and fermented oat milk product.

9. Use of the *B. animalis* strain according to claim 1 in the preparation of a medicament for defecation.

10. Use of the *B. animalis* strain according to claim 1 in the production of extracellular polysaccharides, lactic acid and short-chain fatty acids.

11. Use of the *B. animalis* strain according to claim 1 in the preparation of a medicament for improving immunity and maintaining immune balance;
optionally, the *B. animalis* strain is used to produce cytokine, which is tumor necrosis factor-$\alpha$ (TNF-$\alpha$) and interleukin-6 (IL-6).

12. The use according to claim 11, wherein the single-strain powder of *B. animalis* is used to enhance mouse cellular immune function and monocyte-macrophage function.

13. A lyophilized preparation of *B. animalis* strain, comprising the *B. animalis* strain according to claim 1 and a lyoprotectant.

14. The lyophilized preparation of *B. animalis* strain according to claim 12, wherein the lyoprotectant comprises: 100-150 g/L of skim milk powder, 20-50 g/L of sucrose, 1-2 g/L of vitamin C, and 0.5-1 g/L of L-sodium glutamate.

15. A method for preparing the lyophilized preparation of *B. animalis* strain according to claim 13, comprising the following steps:

(1) strain culture: inoculating the *B. animalis* strain into a sterile liquid medium at an inoculation amount of 1-3% of the total amount of the medium, culturing for 16-24 hours to obtain a seed culture solution, then inoculating the obtained seed culture solution into a fermentation medium at an inoculation amount of 1-3% of the total amount of the medium, and performing fermentation culture to obtain a fermentation broth of the *B. animalis* strain; and
(2) drying the fermentation broth.

16. The method according to claim 15, wherein the fermentation medium comprises: 40-60 g/L of glucose, 60-100 g/L of yeast extract, 3-10 g/L of sodium acetate trihydrate, 0.1-0.2 g/L of magnesium sulfate, 0.05-0.1 g/L of manganese sulfate, 1-2 g/L of dipotassium hydrogen phosphate, 2-4 g/L of triammonium citrate, 1-2 g/L of Tween 80, 0.05-0.1 g/L of calcium chloride, and 0.5-1 g/L of L-cysteine salt.

17. The method according to claim 15, wherein during the fermentation, the sodium hydroxide solution is automatically added to maintain a constant pH of 5.5-6.5 to cause the fermentation until the acid production is stopped, and the fermentation is terminated when the sodium hydroxide is no longer added.

18. The method according to claim 15, wherein the preparation is a live bacteria preparation, and the method further comprises preparing a lyoprotectant after the strain culturing step and before the drying step, and freeze-drying the lyoprotectant in the drying step; preferably, the lyoprotectant used comprises: 80-100 g/L of skim milk powder, 40-50 g/L of trehalose, 2-3 g/L of vitamin C, 4-5 g/L of L-sodium glutamate; preferably, the freeze-drying conditions are: the pre-freezing temperature is -40 ~ -45°C, the pre-freezing time is 4 ~ 5 h, the primary drying temperature is -20 ~ -15°C, the primary drying time is 20 ~ 25 h, the secondary drying temperature is 30 ~ 35°C, and the secondary drying time is 6 ~ 10 h.

19. The method according to claim 15, wherein the preparation is a dead bacteria preparation, and the drying step comprising: firstly, performing heat inactivation, and then drying through a spray drying tower to obtain the dead bacteria preparation; optionally, the heat inactivation condition is 80 ~ 100°C for 10 ~ 40 min.

FIG. 1

FIG. 2

FIG. 3

FIG. 4

FIG. 5

FIG. 6

FIG. 7

FIG. 8

## INTERNATIONAL SEARCH REPORT

| International application No. |
|---|
| **PCT/CN2024/100538** |

| A. | CLASSIFICATION OF SUBJECT MATTER |
|---|---|

C12N1/20(2006.01)i; A23L33/135(2016.01)i; A61K35/745(2015.01)i; A61P1/10(2006.01)i; C12P19/04(2006.01)i; C12R1/01(2006.01)i

According to International Patent Classification (IPC) or to both national classification and IPC

| B. | FIELDS SEARCHED |
|---|---|

Minimum documentation searched (classification system followed by classification symbols)

IPC:C12N,A23L,A61K,A61P,C12P,C12R

Documentation searched other than minimum documentation to the extent that such documents are included in the fields searched

Electronic data base consulted during the international search (name of data base and, where practicable, search terms used)

CNABS, CNTXT, DWPI, ENTXT, ENTXTC, OETXT, VEN, WPABS, WPABSC, CNKI, ISI WEB OF SCIENCE, PUBMED, 万方数据库, WANFANG, GenBank, EMBL: 单核, 动物双歧杆菌, 巨噬, 免疫, IL6, immu+, Bifidobacterium animalis, 白细胞介素, 白介素, IL-6, 肿瘤坏死因子, TNF-αCGMCCNo.25681, SEQ ID NO: 1

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 117327603 A (KELI CO., LTD.) 02 January 2024 (2024-01-02)<br>claims 1-12 | 1-19 |
| PX | CN 117327607 A (KELI CO., LTD.) 02 January 2024 (2024-01-02)<br>claims 1-10 | 1-19 |
| A | US 2021213075 A1 (HUAZHONG AGRICULTURAL UNIVERSITY) 15 July 2021<br>(2021-07-15)<br>description, paragraphs 8-27, and embodiment 3 | 1-19 |
| A | CN 101260377 A (INNER MONGOLIA MENGNIU DAIRY (GROUP) CO., LTD.) 10<br>September 2008 (2008-09-10)<br>claims 1-10 | 1-19 |
| A | CN 113403225 A (QINGDAO NORSON BIOTECHNOLOGY CO., LTD.) 17 September<br>2021 (2021-09-17)<br>description, paragraphs [0002]-[0017] | 1-19 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **18 September 2024** | **26 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/100538** |

## C.    DOCUMENTS CONSIDERED TO BE RELEVANT

| Category* | Citation of document, with indication, where appropriate, of the relevant passages | Relevant to claim No. |
| --- | --- | --- |
| A | CN 116121128 A (SHENZHEN PORSHEALTH BIOENGINEERING CO., LTD.) 16 May 2023 (2023-05-16)<br>    abstract, and claims 1-9 | 1-19 |
| A | KR 20170055093 A (REPUBLIC OF KOREA (MANAGEMENT: RURAL DEVELOPMENT ADMINISTRATION) et al.) 19 May 2017 (2017-05-19)<br>    description, paragraphs 16-60 | 1-19 |
| A | 彭晨芮等 (PENG, Chenrui et al.). "动物双歧杆菌乳亚种BB-12与健康 (Bifidobacterium Animalis Subsp.Lactis Bb-12 and Health: A Systematic Review)"<br>营养学报 *(Acta Nutrimenta Sinica)*, Vol. 45, No. 2, 30 April 2023 (2023-04-30),<br>    pages 120-126 | 1-19 |
| A | CHENG, Jing et al. "Bifidobacterium animalis subsp. lactis HN019 Effects on Gut Health: A Review"<br>*Frontiers in Nutrition*, Vol. 8, 14 December 2021 (2021-12-14),<br>    document number: 790681 | 1-19 |
| A | PEAK, N. S. et al. "Bifidobacterium animalis subsp. lactis strain MG4589 16S ribosomal RNA gene, partial sequence"<br>*GenBank*, 25 March 2020 (2020-03-25),<br>    MT225594.1 | 1-19 |

Form PCT/ISA/210 (second sheet) (July 2022)

**INTERNATIONAL SEARCH REPORT**

International application No.

**PCT/CN2024/100538**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2024/100538**

| Patent document cited in search report | | | Publication date (day/month/year) | Patent family member(s) | | | Publication date (day/month/year) |
|---|---|---|---|---|---|---|---|
| CN | 117327603 | A | 02 January 2024 | None | | | |
| CN | 117327607 | A | 02 January 2024 | None | | | |
| US | 2021213075 | A1 | 15 July 2021 | US | 11786566 | B2 | 17 October 2023 |
| CN | 101260377 | A | 10 September 2008 | None | | | |
| CN | 113403225 | A | 17 September 2021 | None | | | |
| CN | 116121128 | A | 16 May 2023 | None | | | |
| KR | 20170055093 | A | 19 May 2017 | None | | | |

Form PCT/ISA/210 (patent family annex) (July 2022)

**REFERENCES CITED IN THE DESCRIPTION**

**Patent documents cited in the description**

- CN 202310744238
- CN 202310744483